# FASCICULE DE BREVET EUROPEEN

(11) **EP 1 123 653 B1**
(45) Date de publication et mention de la délivrance du brevet: **17.12.2003**
(21) Numéro de dépôt: 01400020.2
(22) Date de dépôt: 05.01.2001
(51) Int. Cl.: A01N 37/46

(54) **Utilisation de dérivés de polyaminoacides comme agents conservateurs**
Verwendung von Polyaminosäurederivate als Konservierungsmittel
Use of polyamino acid derivatives as preservatives

(30) Priorité: 28.01.2000 FR 0001208
(43) Date de publication de la demande: 16.08.2001
(73) Titulaire: L'OREAL, 75008 Paris (FR)
(72) Inventeur: Philippe, Michel, 91320 Wissous (FR); Benard, Sylvie, 95570 Attainville (FR); Cupferman, Sylvie, 94240 l'Hay les Roses (FR)
(74) Mandataire: Le Blainvaux Bellegarde, Françoise

(56) Documents cités:
- EP-A- 0 884 058
- WO-A-92/20647
- WO-A-95/00547
- FR-A- 2 776 510

## Description

La présente invention a trait à l'utilisation de certains dérivés de polyaminoacides comme agents conservateurs dans des compositions cosmétiques ou dermatologiques, notamment destinées à une application topique.

Il est courant d'introduire dans les compositions cosmétiques ou dermatologiques, des conservateurs chimiques destinés à lutter contre le développement des microorganismes dans ces compositions, ce qui les rendrait rapidement inaptes à l'utilisation. Il faut notamment protéger les compositions contre les microorganismes susceptibles de se développer à l'intérieur de la composition et également contre ceux que l'utilisateur pourrait y introduire en la manipulant, en particulier lors de la préhension avec les doigts de produits en pot. Des conservateurs chimiques couramment utilisés sont notamment les parabènes ou les composés libérateurs de formol. Ces conservateurs présentent toutefois l'inconvénient de causer des irritations, en particulier sur les peaux sensibles, lorsqu'ils sont présents à des taux relativement importants. Comme autres conservateurs connus, on peut citer les hydroxyacides organiques. Mais ces composés peuvent également générer des irritations du fait de leur effet desquamant sur la peau, ce qui n'est pas toujours bien toléré.

On connaît par EP884058 une solution comprenant de la polylysine pour désinfecter les lentilles de contact.
On connaît également par WO92/20647 un procédé de préparation de lipoaminoacides par hydrolyse de protéines et acylation.
On connaît encore par WO95/00547 une composition antimicrobienne comprenant un polymère et un oligopeptide particulier antimicrobien.

Il subsiste donc le besoin d'agents conservateurs, notamment d'agents anti-microbiens, ayant une action au moins aussi efficace que les composés de l'art antérieur, mais n'en présentant pas les inconvénients.
Par ailleurs, il serait souhaitable de disposer d'agents anti-microbiens présentant un spectre anti-microbien au moins aussi large, voire supérieur, à ceux des composés déjà existants.
La présente invention a pour but de proposer de nouveaux agents conservateurs, présentant notamment un spectre anti-microbien large, et ne présentant pas les inconvénients de l'art antérieur.

Ainsi, un objet de la présente invention est donc un procédé de conservation d'une composition cosmétique ou pharmaceutique, caractérisé en ce qu'il consiste à incorporer dans ladite composition au moins un dérivé de polyaminoacide de formule (I) tel que défini ci-après.

On a donc constaté que, de manière surprenante et inattendue, certains dérivés de polyaminoacides présentaient de bonnes propriétés anti-microbiennes, que cela soit vis-à-vis des virus, des bactéries, des levures ou des champignons/moisissures. De par leur spectre anti-microbien large, ces dérivés peuvent donc être employés, notamment dans les compositions cosmétiques, comme seuls agents anti-microbiens, notamment comme agents anti-bactériens, comme agents anti-viraux, comme agents anti-levures et/ou comme agents antifongiques.
Ces dérivés de polyaminoacides peuvent être employés de manière avantageuse dans les compositions cosmétiques ou pharmaceutiques, notamment dermatologiques, en tant qu'agent conservateur.
Par ailleurs, les composés selon l'invention présentent comme avantage, une structure chimique clairement définie et caractérisée, d'où une reproductibilité de leur fabrication aisée; leur faisabilité industrielle est également assez simple. De plus, ils possèdent une bonne solubilité ou compatibilité avec les milieux couramment employés en cosmétique, les milieux aqueux notamment.

Les dérivés de polyaminoacides utilisés dans le cadre de la présente invention sont bien connus dans l'art antérieur, notamment dans le domaine cosmétique, en particulier pour leurs propriétés d'hydratation, ou pour leur application en capillaire.
On peut ainsi citer la demande japonaise JP-07/041467 qui décrit une classe de polyaminoacides de poids moléculaire élevé constitué essentiellement de cystéine, ainsi que le procédé de préparation de ces polyaminoacides.
Une classe de polyaminoacides caractérisée par la présence de fonctions thiols et/ou disulfures a également été décrite dans la demande japonaise JP-06/248072. Ces polyaminoacides réagissent avec les liaisons thiols de la kératine formant ainsi des ponts disulfures, ce qui permet de rehausser les qualités de brillance et de coloration de la chevelure.
Des polyaminoacides constitués essentiellement d'acides aminés à chaînes neutres et acides ont été décrits dans la demande japonaise JP-04/198114, ainsi que leur utilisation en tant qu'agent hydratant.
On peut encore citer la demande FR2776510 qui décrit une composition cosmétique destinée au renforcement ou au soin des fibres kératiniques, notamment capillaire, comprenant des dérivés de polyamino-acides.

Les dérivés de polyaminoacides employés dans le cadre de la présente invention répondent à la formule (I) suivante : 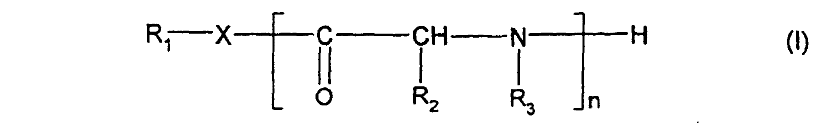 dans laquelle :
- X représente O, S, NH ou NR" avec R" représentant un radical hydrocarboné, saturé ou insaturé, linéaire ou ramifié, en C₁₋₆;
- R₁ représente
   - un radical hydrocarboné linéaire ou ramifié, saturé ou insaturé, en C₁₋₄₀, éventuellement substitué par au moins un radical hydroxy et/ou un radical -NRR' et/ou éventuellement interrompu par au moins un hétéroatome choisi parmi N, O ou Si, R et R', identiques ou différents, étant un atome d'hydrogène ou un radical hydrocarboné, saturé ou insaturé, linéaire ou ramifié, en C₁₋₆;
   - un radical 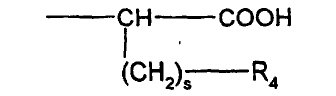 avec s égal à 0, 1, 2, 3 ou 4; et R₄ représentant un atome d'hydrogène ou un radical choisi parmi -NH₂, -OH, -SH, -CHOHCH₃, -CONH₂, -NH-C(NH₂)=NH, -C₆H₅, - C₆H₄OH et 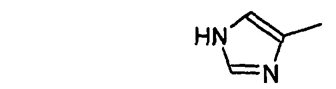
   - un radical 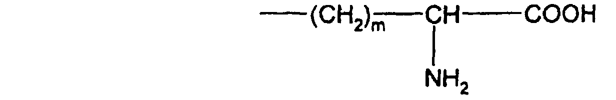 avec m égal à 3, 4 ou 5;
- R₂ représente un atome d'hydrogène; un radical hydrocarboné, saturé ou insaturé, linéaire ou ramifié, en C₁₋₈; un radical choisi parmi -CH₂C₆H₅, -CH₂C₆H₄OH, -CH₂OH, -CHOHCH₃, -(CH₂)ₜ-NH₂ avec t égal à 3 ou 5;
- R₃ représente un atome d'hydrogène ou un radical hydrocarboné, saturé ou insaturé, linéaire ou ramifié, en C₁₋₆;
- n est un nombre moyen supérieur à 1 tel que le poids moléculaire du dérivé de polyaminoacide est compris entre 100 et 200 000;
- l'unité répétitive pouvant être identique ou différente pour un même dérivé, R₂ et/ou R₃ prenant alors au moins l'une des autres significations données pour ces radicaux, de préférence les unités sont identiques.

Les sels, notamment d'acide minéral ou d'acide organique, desdits dérivés de polyaminoacides font également partie de la présente invention.

Selon un mode de réalisation préféré de la présente invention, on emploie au moins un dérivé pour lequel l'on a une des significations suivantes :
- X représente O, S et de préférence NH ou NR" avec R" représentant un radical hydrocarboné, saturé ou insaturé, linéaire ou ramifié, en C₁₋₆;
- R₁ représente un radical hydrocarboné linéaire ou ramifié, saturé ou insaturé, en C₈₋₄₀, éventuellement substitué par au moins un radical hydroxy et/ou un radical
- NRR' et/ou éventuellement interrompu par au moins un hétéroatome choisi parmi N, O ou Si, R et R', identiques ou différents, étant un atome d'hydrogène ou un radical hydrocarboné, saturé ou insaturé, linéaire ou ramifié, en C₁₋₆;
- R₂ représente un atome d'hydrogène;
- R₃ représente un radical hydrocarboné saturé, linéaire ou ramifié, en C₁₋₆; notamment un radical méthyle ou éthyle;
- n est compris entre 2 et 100 et/ou est tel que le poids moléculaire du dérivé de polyaminoacide est compris entre 150 et 10 000.

Encore plus préférentiellement, on choisit le composé employé dans le cadre de la présente invention parmi les dérivés dans lesquels :
- X représente O, S et de préférence NH;
- R₁ représente un radical hydrocarboné, linéaire ou ramifié, saturé en C₁₀₋₂₄, éventuellement substitué par 1, 2, 3 ou 4 radicaux hydroxy ; ou un radical hydrocarboné, linéaire ou ramifié, comportant une ou plusieurs insaturations doubles, en C₁₂₋₂₄, éventuellement substitué par au moins un radical hydroxy;
- R₂ représente un atome d'hydrogène;
- R₃ représente un radical méthyle;
- n est compris entre 4 et 50 et/ou est tel que le poids moléculaire du dérivé de polyaminoacide est compris entre 300 et 8 000.

Les dérivés de polyaminoacides selon l'invention peuvent aisément être préparés par l'homme du métier sur base de ses connaissances générales. La demande FR2776510 décrit notamment un procédé permettant la préparation de ces composés.

Les dérivés de polyaminoacides peuvent être présents dans les compositions cosmétiques ou pharmaceutiques en une quantité suffisante pour obtenir l'effet recherché, et notamment comprise entre 0,001 et 30% en poids, de préférence entre 0,01 et 15% en poids, et plus particulièrement entre 0,5 et 5% en poids, par rapport au poids total de la composition.

Les compositions comprenant lesdits dérivés de polyaminoacides comprennent un milieu cosmétiquement ou pharmaceutiquement acceptable, c'est-à-dire compatible avec la peau, les muqueuses, les cheveux et le cuir chevelu. Elles peuvent se présenter sous toutes les formes galéniques appropriées pour une application topique, notamment sous forme de solution aqueuse, hydroalcoolique, organique ou huileuse; de suspension ou de dispersion dans des solvants ou des corps gras, de type lotion ou sérum; sous forme de dispersion vésiculaire; sous forme d'émulsion E/H, H/E ou multiple telle qu'une crème ou un lait; sous forme de pommade, de gel, de bâtonnet solide, de produits anhydres pâteux ou solides, de mousse notamment aérosol ou de spray.
Le milieu physiologiquement acceptable dans lequel les dérivés peuvent être employés, ainsi que ses constituants, leur quantité, la forme galénique de la composition et son mode de préparation, peuvent être choisis par l'homme du métier sur la base de ses connaissances générales en fonction du type de composition recherchée.

Notamment, la composition peut comprendre tout corps gras usuellement utilisé dans le domaine d'application envisagé. On peut notamment citer les corps gras siliconés tels que les huiles, les gommes et les cires de silicone, ainsi que les corps gras non siliconés tels que les huiles et les cires d'origine végétale, minérale, animale et/ou synthétique. Les huiles peuvent éventuellement être volatiles
ou non volatiles. On peut encore citer les hydrocarbures, les esters et les éthers de synthèse, les alcools gras et les acides gras.
La composition peut également comprendre un milieu aqueux, un milieu hydroalcoolique contenant un alcool tel que l'éthanol ou l'isopropanol, ou un milieu organique comprenant des solvants organiques usuels tels que des alcools en C₁₋₆, notamment l'éthanol et l'isopropanol, des glycols tels que le propylène glycol, des cétones.

La composition peut comprendre au moins un émulsionnant classique, choisi parmi les émulsionnants amphotères, anioniques, cationiques ou non ioniques, utilisés seuls ou en mélange.

Elle peut également comprendre les adjuvants habituels dans le domaine considéré, tels que les épaississants ou gélifiants hydrophiles ou lipophiles, les additifs hydrophiles ou lipophiles, les actifs notamment cosmétiques, les conservateurs, les antioxydants, les parfums, les charges, les pigments, les filtres UV, les absorbeurs d'odeur, les colorants, les hydratants (glycérine), des vitamines, des acides gras essentiels, des polymères liposolubles notamment hydrocarbonés, les opacifiants, les stabilisants, les sequestrants, les conditionneurs, les agents propulseurs.
Bien entendu l'homme du métier veillera à choisir ce ou ces éventuels adjuvants complémentaires et/ou leur quantité de manière telle que les propriétés avantageuses de la composition selon l'invention ne soient pas, ou substantiellement pas, altérées par l'adjonction envisagée.

Le pH des compositions selon l'invention est de préférence inférieur à 7, notamment compris entre 3 et 6.

Les dérivés de polyaminoacides employés dans le cadre de la présente invention présentent donc une activité anti-microbienne, notamment une activité anti-bactérienne, anti-virale et anti-fongique, remarquable.
On a constaté que, selon la nature des substituants X, R₁, R₂ et/ou R₃, et/ou la valeur de 'n', il est possible d'obtenir des composés plus ou moins actifs vis-à-vis de tel type de microbe. On peut ainsi moduler le champ d'action des dérivés selon l'invention, en focalisant sur tel microbe, en choisissant de manière adéquate la nature chimique du composé employé.

Ils trouvent donc une application toute particulière dans des compositions pouvant se présenter :
- sous la forme d'un produit de maquillage de la peau du visage, du corps ou des lèvres, tel qu'un fond de teint, un fard à joues ou à paupières, un stick anti-cernes, un stick camouflant, un eye-liner, un mascara, un rouge à lèvres, un vernis-à-ongles, un soin des ongles;
- sous la forme d'un produit de soin dermatologique ou cosmétique de la peau du visage, du corps y compris le cuir chevelu, des lèvres, tel qu'une base de soin pour les lèvres, une base fixante à appliquer sur un rouge à lèvres classique, une composition de protection solaire ou de bronzage artificiel; un produit déodorant; une composition de soin (de jour, de nuit, anti-rides, hydratante, etc.) pour le visage; une composition matifiante pour le visage; un gel ou une crème de nettoyage ou de démaquillage; un lait corporel de protection ou de soin; un lait purifiant;
- sous la forme d'une composition désodorisante; d'un gel ou lotion après-rasage; d'une crème dépilatoire;
- sous la forme d'une composition pharmaceutique;
- sous la forme d'une composition solide telle qu'un savon ou un pain de nettoyage;
- sous la forme d'une composition pour aérosol comprenant également un agent propulseur sous pression.;
- sous la forme d'une composition pour soins capillaires, et notamment un shampooing, une lotion de mise en plis, une lotion traitante, une crème ou un gel coiffant, une composition de teinture (notamment d'oxydation) éventuellement sous forme de shampooing colorant, de lotion restructurante pour cheveux, une composition de permanente, une lotion ou un gel antichute, un shampooing antiparasitaire; une lotion ou un shampooing anti-pelliculaire; un shampoing traitant notamment anti-séborrhéique.
- sous la forme d'une composition à usage bucco-dentaire, par exemple une pâte dentifrice.

La présente invention est illustrée plus en détails dans les exemples suivants.

### Exemple 1

Préparation du composé de formule (I) avec R₁ = C₁₅H₃₁CH(OH)-CH(CH₂OH)-,

X = -NH-, R₂ = H, R₃ = -CH₃ et n = 14,2.

Dans un réacteur de 1 litre, on met en suspension 46 g (0,4 mole) de N-carboxy-anhydride sarcosine dans 250 ml de toluène sous atmosphère d'azote. On ajoute, par petites fractions, une suspension de 8,2 g (0,027 mole) de (D/L, érythro-thréo) 2-amino octadécane-1,3 diol dans 250 ml de toluène. Après la fin de l'addition, on porte le mélange réactionnel à 80°C pendant environ 3 heures. On laisse ensuite refroidir à température ambiante et on ajoute 200 ml d'éthanol (98°C) pour solubiliser le milieu.
Après évaporation des solvants sous pression réduite et séchage sous vide, on obtient 34,5 g d'une poudre de couleur marron.
L'indice "n" a été déterminé par RMN.

Selon le même mode opératoire que ci-dessus en faisant varier la proportion de (D/L) 2-amino octadécane-1,3 diol, on a obtenu des dérivés de polyaminoacides ayant la même structure mais ayant les indices "n" suivants :
- exemple 1(a) n = 9,8
- exemple 1(b) n = 7,6

### Exemple 2

Préparation du composé de formule (I) avec R₁ = C₁₀H₂₁-CH(C₈H₁₇)-CH₂-, R₂ = H, X = -NH-, R₃ = -CH₃ et n = 14.

Dans un réacteur de 1 litre, on met en suspension 46 g (0,4 mole) de N-carboxy-anhydride sarcosine dans 500 ml de toluène. On ajoute ensuite, goutte à goutte, 8,1 g (0,027 mole) de 2-octyl dodécylamine. Après la fin de l'addition, on porte le mélange à 80°C pendant environ 2 heures. On laisse ensuite refroidir à température ambiante puis ajoute 50 ml d'éthanol (98°C).
Après évaporation des solvants sous pression réduite et séchage sous vide, on obtient 36,7 g d'une poudre de couleur marron. L'indice "n" a été déterminé par RMN.

Selon le même mode opératoire que ci-dessus, mais en faisant varier la proportion de 2-octyl dodécylamine, on a obtenu des dérivés de polyaminoacides ayant la même structure mais ayant les indices "n" suivants :
- exemple 2(a) n = 9,6
- exemple 2(b) n = 7,4

### Exemple 3

Préparation du composé de formule (I) avec R₁ = C₁₈H₃₃-, X = -NH-, R₂ = H, R₃ = -CH₃ et n = 7,2.

Ce polyaminoacide est obtenu selon le même mode opératoire que celui décrit à l'exemple 2 mais en faisant réagir 12 g (0,05 mole) d'hexadécylamine avec la N-carboxyanhydride sarcosine.
Après évaporation des solvants et séchage sous vide, on obtient 40 g d'une poudre.
L'indice "n" a été déterminé par RMN.

En faisant varier la proportion d'hexadécylamine, on a obtenu les dérivés de polyaminoacides ayant la même structure mais ayant les indices "n" suivants :
- exemple 3(a) n = 9,2
- exemple 3(b) n = 12,5

### Exemple 4

Préparation du composé de formule (I) avec R₁ = C₈H₁₇-CH=CH-C₈H₁₆-, X = -NH-, R₂ = H, R₃ = -CH₃ et n = 7,2.

Ce composé est obtenu selon le même mode opératoire que celui décrit à l'exemple 2 mais en faisant réagir 13 g (0,05 mole) d'oléylamine avec la N-carboxy-anhydride sarcosine.
Après évaporation des solvants et séchage sous vide, on obtient 42 g d'une poudre.
L'indice "n" a été déterminé par RMN.

En faisant varier la proportion d'oléylamine, on a obtenu les dérivés de polyaminoacides ayant la même structure mais ayant les indices "n" suivants :
- exemple 4(a) n = 10, 5
- exemple 4(b) n = 13,2

### Exemple 5

On a déterminé l'activité anti-microbienne du composé préparé à l'exemple 4, à différentes concentrations de matière active (M.A.).

Cette activité a été regardée vis-à-vis de 2 bactéries gram- (*E*. *coli* et P. aeruginosa), d'une bactérie gram+ (*E*. *faecalis*), d'une levure (*C*. *albicans*) et d'une moisissure (*A*. *niger*).

Les étapes pour réaliser ce test sont les suivantes :
1/ culture du microorganisme : les bactéries sont cultivées sur gélose Tryptocaséine soja en pente; la levure sur gélose Sabouraud en pente et la moisissure sur gélose au malt.
2/ préparation de l'inoculum : pour les bactéries et la levure : 24 heures avant le début du test, on effectue un repiquage de la souche et on laisse incuber pendant 24 heures à 35°C; pour la moisissure : 5 jours. A l'issue de la période d'incubation, on lave la pente avec 9 ml de diluant approprié. La suspension obtenue titre à 10⁸ germes/ml.
3/ préparation de l'échantillon : on dépose dans un flacon de verre appelé pilulier, 20 g de la composition comprenant le composé à tester et 0,2 ml d'inoculum (soit 10⁶ germes/ml), on homogénéise et on laisse incuber à 22°C, dans l'obscurité, pendant 7 jours. Parallèlement, on prépare un témoin (placebo) pour vérifier que les germes sont dans des conditions de croissance favorables pendant la durée du test.
4/ prélèvements et dénombrements : après 7 jours de contact, on homogénéise le contenu du pilulier et on en prélève 1 g. Après avoir déterminé la dilution appropriée pour pouvoir effectuer le comptage, on étale cette dilution en surface de boîtes de Pétri gélosées (milieu Eugon LT100), puis on laisse incuber les boîtes de Pétri pendant 24 heures à 5 jours, selon les microorganismes, à l'étuve à 35°C. Puis, on effectue le comptage des colonies sur les boîtes contenant plus de 20 et moins de 200 colonies.

La composition testée consiste en une solution aqueuse (pH 7) du composé de l'exemple 4(a).

Les résultats obtenus sont indiqués dans le tableau suivant. Ils sont exprimés en nombre de micro-organismes par gramme de préparation :

| Composé testé | *E.* coli | *P.* aeruginosa | *E.* faecalis | *C.* albicans | *A.* niger |
|---|---|---|---|---|---|
| composé à 0,5% M.A. | < 200 | < 200 | < 200 | < 200 | < 200 |
| composé à 0,05% M.A. | < 200 | < 200 | < 200 | < 200 | 26 000 |
| témoin | 3,1.10⁶ | 2,2.10⁸ | 3,1.10⁶ | 2,4.10⁶ | 8,0.10⁵ |

On constate donc que le composé testé présente un spectre anti-microbien très large.

### Exemple 6 : Gel pour le visage

- Polyacrylate de glycéryle (Norgel) 30%
- Polyacrylamide/C13-14 Isoparaffine/laureth-7 (Sepigel 305) 2%
- Huile de silicone 10%
- Composé de l'exemple 1a 5%
- Eau qsp 100%

### Exemple 7 : Lotion

- composé de l'exemple 2a 0,2%
- glycérine 2%
- alcool éthylique 20%
- butanol oxyéthyléné (260E) oxypropyléné (260P), huile de ricin hydrogénée oxyéthylénée (40 OE) dans l'eau 1%
- eau déminéralisée qsp 100%

### Exemple 8 : Crème nettoyante moussante

- monostéarate d'éthylène glycol 2%
- composé de l'exemple 3a 0,5%
- silicate de magnésium et d'aluminium hydraté 1,7%
- hydroxypropylméthylcellulose 0,8%
- mélange de cocoyl isethionate de sodium et d'acides gras de coprah (Elfan AT 84 G de Akzo) 15%
- acide stéarique 1,25%
- lauroyl sarcosinate de sodium à 30 % dans l'eau 10%
- parfum qs
- eau déminéralisée qsp 100%

### Exemple 9 : Crème de soin

- tristéarate de sorbitane 1 %
- composé de l'exemple 4a 1,5%
- homopolymère carboxyvinylique réticulé 0,4%
- gomme de xanthane 0,5%
- copolymère diméthacrylate d'éthylèneglycol/méthacrylaet de lauryle 1%
- cyclopentadiméthylsiloxane 6%
- glycérine 3%
- émulsionnant 4%
- parfum qs
- eau déminéralisée qsp 100%

### Exemple 10 : Gel traitant

- composé de l'exemple 1b 1 %
- gomme de xanthane 1%
- glycérine 2%
- éthanol 20%
- mélange alcool butylique oxyéthyléné (260E) oxypropyléné (26OP), huile de ricin hydrogénée oxyéthylénée (40OE) dans l'eau 1%
- parfum qs
- eau déminéralisée qsp 100%

### Exemple 11 : Crème teintée

- lécithine hydrogénée 2,4%
- huile d'amandes d'abricot 6%
- copolymère diméthacrylate d'éthylèneglycol/méthacrylaet de lauryle 1%
- stérols de soja oxyéthylénés (5 OE) 1,6%
- composé de l'exemple 2b 1%
- oxydes de fer 0,9%
- oxyde de titane 5%
- polyacrylamide / C₁₃-C₁₄-Isoparaffine / Laureth-7 (Sepigel 305) 4%
- cyclopentadiméthyisiloxane 6%
- glycérine 6%
- propylène glycol 6%
- parfum qs
- eau déminéralisée qsp 100%

### Exemple 12 : Stick camouflant

- cires (Carnauba et Ozokérite) 14%
- fraction liquide de beuyrre de karité 4%
- oxydes de titane et de zinc 22%
- oxydes de fer 4%
- composé de l'exemple 3b 1 %
- polydiméthylsiloxane / silice hydratée 0,1 %
- alcool cétylique 1,4%
- isoparaffine qsp 100%

## Revendications

1. Procédé de conservation d'une composition cosmétique ou pharmaceutique, **caractérisé en ce qu'**il consiste à incorporer dans ladite composition au moins un dérivé de polyaminoacide répondant à la formule (I) : 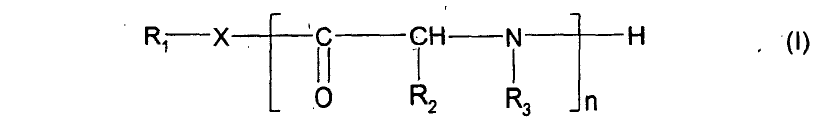 dans laquelle :
- X représente O, S, NH ou NR" avec R" représentant un radical hydrocarboné, saturé ou insaturé, linéaire ou ramifié, en C₁₋₆;
- R₁ représente
- un radical hydrocarboné linéaire ou ramifié, saturé ou. insaturé, en C₁₋₄₀, éventuellement substitué par au moins un radical hydroxy et/ou un radical -NRR' et/ou éventuellement interrompu par au moins un hétéroatome choisi parmi N, O ou Si, R et R', identiques ou différents, étant un atome d'hydrogène ou un radical hydrocarboné, saturé ou insaturé, linéaire ou ramifié, en C₁₋₆;
- un radical 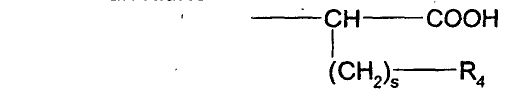 avec s égal à 0, 1, 2, 3 ou 4; et R₄ représentant un atome d'hydrogène ou un radical choisi parmi -NH₂, -OH, -SH, -CHOHCH₃, -CONH₂, -NH-C(NH₂)=NH, -C₆H₅, -C₆H₄OH et 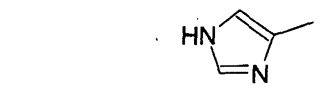
- un radical 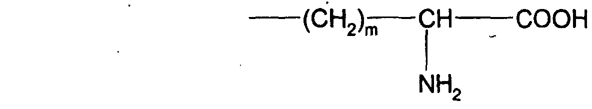 avec m égal à 3, 4 ou 5;
- R₂ représente un atome d'hydrogène; un radical hydrocarboné, saturé ou insaturé, linéaire ou ramifié, en C₁₋₈; un radical choisi parmi -CH₂C₆H₅, -CH₂C₆H₄OH, -CH₂OH, -CHOHCH₃, -(CH₂)ₜ-NH₂ avec t égal à 3 ou 5;
- R₃ représente un atome d'hydrogène ou un radical hydrocarboné, saturé ou insaturé, linéaire ou ramifié, en C₁₋₆;
- n est un nombre moyen supérieur à 1 tel que le poids moléculaire du dérivé de polyaminoacide est compris entre 100 et 200 000;
- l'unité répétitive pouvant être identique ou différente pour un même dérivé, R₂ et/ou R₃ prenant alors au moins l'une des autres significations données pour ces radicaux,
ou d'un de ses sels.

2. Procédé selon la revendication 1, dans lequel le dérivé de polyaminoacide répond à la formule (I) dans laquelle :
- X représente O, S, NH ou NR" avec R" représentant un radical hydrocarboné, saturé ou insaturé, linéaire ou ramifié, en C₁₋₆;
- R₁ représente un radical hydrocarboné linéaire ou ramifié, saturé ou insaturé, en C₈₋₄₀, éventuellement substitué par au moins un radical hydroxy et/ou un radical
- NRR' et/ou éventuellement interrompu par au moins un hétéroatome choisi parmi N, O ou Si, R et R', identiques ou différents, étant un atome d'hydrogène ou un radical hydrocarboné, saturé ou insaturé, linéaire ou ramifié, en C₁₋₆;
- R₂ représente un atome d'hydrogène;
- R₃ représente un radical hydrocarboné saturé, linéaire ou ramifié, en C₁₋₆; notamment un radical méthyle ou éthyle; et/ou
- n est compris entre 2 et 100 et/ou est tel que le poids moléculaire du dérivé de polyaminoacide est compris entre 150 et 10 000.

3. Procédé selon l'une des revendications précédentes, dans lequel le dérivé de polyaminoacide répond à la formule (I) dans laquelle :
- X représente NH;
- R₁ représente un radical hydrocarboné, linéaire ou ramifié, saturé en C₁₀₋₂₄, éventuellement substitué par 1, 2, 3 ou 4 radicaux hydroxy ; ou un radical hydrocarboné, linéaire ou ramifié, comportant une ou plusieurs insaturations doubles, en C₁₂₋₂₄, éventuellement substitué par au moins un radical hydroxy;
- R₂ représente un atome d'hydrogène;
- R₃ représente un radical méthyle; et/ou
- n est compris entre 4 et 50 et/ou est tel que le poids moléculaire du dérivé de polyaminoacide est compris entre 300 et 8 000.

4. Procédé selon l'une des revendications précédentes, dans lequel la composition cosmétique ou pharmaceutique comprend par ailleurs un milieu physiologiquement acceptable.

5. Procédé selon l'une des revendications précédentes, dans lequel le dérivé de polyaminoacides est présent dans la composition cosmétique ou pharmaceutique en une quantité comprise entre 0,001 et 30% en poids, de préférence entre 0,01 et 15% en poids, et plus particulièrement entre 0,5 et 5% en poids, par rapport au poids total de la composition.

6. Procédé selon l'une des revendications précédentes, dans lequel la composition cosmétique ou pharmaceutique se présente :
- sous la forme d'un produit de maquillage de la peau du visage, du corps ou des lèvres, tel qu'un fond de teint, un fard à joues ou à paupières, un stick anti-cemes, un stick camouflant, un eye-liner, un mascara, un rouge à lèvres, un vernis-à-ongles, un soin des ongles;
- sous la forme d'un produit de soin dermatologique ou cosmétique de la peau du visage, du corps y compris le cuir chevelu, des lèvres, tel qu'une base de soin pour les lèvres, une base fixante à appliquer sur un rouge à lèvres classique, une composition de protection solaire ou de bronzage artificiel; un produit déodorant; une composition de soin (de jour, de nuit, anti-rides, hydratante, etc.) pour le visage; une composition matifiante pour le visage; un gel ou une crème de nettoyage ou de démaquillage; un lait corporel de protection ou de soin; un lait purifiant;
- sous la forme d'une composition désodorisante; d'un gel ou lotion après-rasage; d'une crème dépilatoire;
- sous la forme d'une composition pharmaceutique;
- sous la forme d'une composition solide telle qu'un savon ou un pain de nettoyage;
- sous la forme d'une composition pour aérosol comprenant également un agent propulseur sous pression.;
- sous la forme d'une composition pour soins capillaires, et notamment un shampooing, une lotion de mise en plis, une lotion traitante, une crème ou un gel coiffant, une composition de teinture (notamment d'oxydation) éventuellement sous forme de shampooing colorant, de lotion restructurante pour cheveux, une composition de permanente, une lotion ou un gel antichute, un shampooing antiparasitaire; une lotion ou un shampooing anti-pelliculaire; un shampoing traitant notamment anti-séborrhéique.
- sous la forme d'une composition à usage bucco-dentaire, par exemple une pâte dentifrice.

## Patentansprüche

1. Verfahren zur Konservierung einer kosmetischen oder pharmazeutischen Zusammensetzung, **dadurch gekennzeichnet, dass** es darin besteht, in die Zusammensetzung mindestens ein Polyaminosäurederivat der Formel (I) einzuarbeiten: 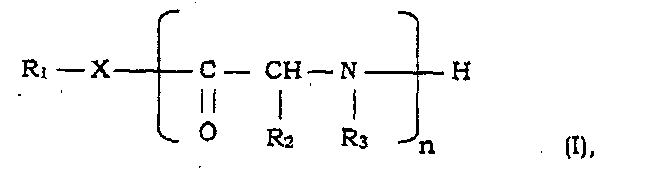 worin bedeuten:
- X O, S, NH oder NR", wobei R" eine gesättigte oder ungesättigte, geradkettige oder verzweigte Kohlenwasserstoffgruppe mit. 1 bis 6 Kohlenstoffatomen ist;
- R₁
- eine geradkettige oder verzweigte, gesättigte oder ungesättigte Kohlenwasserstoffgruppe mit 1 bis 40 Kohlenstoffatomen, die gegebenenfalls mit mindestens einer Hydroxygruppe und/oder einer Gruppe -NRR' substituiert und/oder gegebenenfalls durch mindestens ein Heteroatom, das unter N, O oder Si ausgewählt ist, unterbrochen ist, wobei die Gruppen R und R', die gleich oder verschieden sind, ein Wasserstoffatom oder eine gesättigte oder ungesättigte, geradkettige oder verzweigte Kohlenwasserstoffgruppe mit 1 bis 6 Kohlenstoffatomen bedeuten;
- eine Gruppe 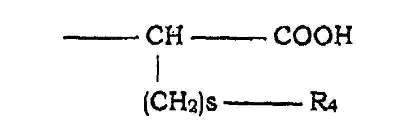 wobei s 0, 1, 2, 3 oder 4 bedeutet; und die Gruppe R₄ Wasserstoff oder eine Gruppe bedeutet, die unter den folgenden Gruppen ausgewählt ist: -NH₂, -OH, -SH, -CHOHCH₃, - CONH₂, -NH-C(NH₂)=NH, -C₆H₅, -C₆H₄OH und 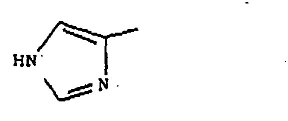
- eine Gruppe 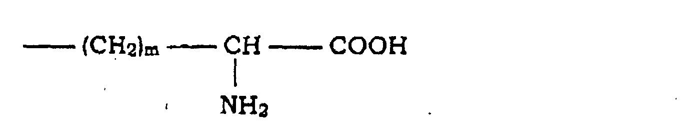
wobei m 3, 4 oder 5 bedeutet;
- R₂ ein Wasserstoffatom, eine gesättigte oder ungesättigte, geradkettige oder verzweigte Kohlenwasserstoffgruppe mit 1 bis 8 Kohlenstoffatomen; eine Gruppe, die unter -CH₂C₆H₅, -CH₂C₆H₄OH, -CH₂OH, -CHOHCH₃ und -(CH₂)ₜ-NH₂ mit t = 3 oder 5 ausgewählt ist;
- R₃ ein Wasserstoffatom oder eine gesättigte oder ungesättigte, geradkettige oder verzweigte Kohlenwasserstoffgruppe mit 1 bis 6 Kohlenstoffatomen;
- n einen solchen Mittelwert über 1, dass die Molmasse des Polyaminosäurederivats im Bereich von 100 bis 200 000 liegt;
- wobei die wiederkehrende Einheit in einem Derivat gleich oder verschieden sein kann, wobei die Gruppe R₂ und/ oder die Gruppe R₃ dann mindestens eine der anderen für diese Gruppen angegebenen Bedeutungen annimmt;
oder ein Salz dieser Verbindungen.

2. Verfahren nach Anspruch 1, wobei das Polyaminosäurederivat der Formel (I) entspricht, worin:
- X O, S, NH oder NR" bedeutet, wobei R" eine gesättigte oder ungesättigte, geradkettige oder verzweigte C₁₋₆-Kohlenwasserstoffgruppe ist;
- R₁ eine geradkettige oder verzweigte, gesättigte oder ungesättigte C₈₋₄₀-Kohlenwasserstoffgruppe bedeutet, die gegebenenfalls mit mindestens einer Hydroxygruppe und/oder einer Gruppe -NRR' substituiert und/oder gegebenenfalls durch mindestens ein Heteroatom, das unter N, O oder Si ausgewählt ist, unterbrochen ist, wobei R und R', die identisch oder voneinander verschieden sind, ein Wasserstoffatom oder eine gesättigte oder ungesättigte, geradkettige oder verzweigte Ci-6-Kohlenwasserstoffgruppe bedeuten;
- R₂ ein Wasserstoffatom bedeutet;
- R₃ eine gesättigte, geradkettige oder verzweigte C₁₋₆-Kohlenwasserstoffgruppe und insbesondere Methyl oder Ethyl bedeutet; und/oder
- n im Bereich von 2 bis 100 liegt und/oder so gewählt ist, dass das Molekulargewicht des Polyaminosäurederivats im Bereich von 150 bis 10 000 liegt.

3. Verfahren nach einem der vorhergehenden Ansprüche, wobei das Polyaminosäurederivat der Formel (I) entspricht, worin:
- X NH bedeutet;
- R₁ eine geradkettige oder verzweigte, gesättigte C₁₀₋₂₄-Kohlenwasserstoffgruppe bedeutet, die gegebenenfalls mit 1, 2, 3 oder 4 Hydroxygruppen substituiert ist; oder eine geradkettige oder verzweigte C₁₂₋₂₄-Kohlenwasserstoffgruppe, die eine oder mehrere Doppelbindungen enthält und gegebenenfalls mit mindestens einer Hydroxygruppe substituiert ist;
- R₂ ein Wasserstoffatom bedeutet;
- R₃ eine Methylgruppe bedeutet; und/oder
- n im Bereich von 4 bis 50 liegt und/oder so gewählt ist, dass das Molekulargewicht des Polyaminosäurederivats im Bereich von 300 bis 8 000 liegt.

4. Verfahren nach einem der vorhergehenden Ansprüche, wobei die kosmetische oder pharmazeutische Zusammensetzung ferner ein physiologisch akzeptables Medium enthält.

5. Verfahren nach einem der vorhergehenden Ansprüche, wobei das Polyaminosäurederivat in der kosmetischen oder pharmazeutischen Zusammensetzung in einer Menge von 0,001 bis 30 Gew.-%, vorzugsweise 0,01 bis 15 Gew.-% und insbesondere 0,5 bis 5 Gew.-%, bezogen auf das Gesamtgewicht der Zusammensetzung, enthalten ist.

6. Verfahren nach einem der vorhergehenden Ansprüche, wobei die kosmetische oder pharmazeutische Zusammensetzung vorliegt als:
- Produkt zum Schminken der Gesichtshaut, der Haut des Körpers oder der Lippen, wie beispielsweise als Make-up, Wangenrouge, Lidschatten, Produkt gegen Augenringe, Abdeckstift, Eyeliner, Mascara, Lippenstift, Nagellack oder Nagelpflegeprodukt;
- dermatologisches oder kosmetisches Produkt zur Pflege der Gesichtshaut, der Haut des Körpers einschließlich der Kopfhaut und der Lippen, beispielsweise als Lippenpflegemittel, fixierende Masse, die auf einen herkömmlichen Lippenstift aufgebracht wird, Zusammensetzung zum Sonnenschutz oder zur künstlichen Bräunung, desodorisierendes Produkt; Zusammensetzung zur Pflege des Gesichts (tagsüber zu verwenden, für die Nacht, gegen Falten, hydratisierend und dergleichen); mattierende Zusammensetzung für das Gesicht; Gel oder Creme zur Reinigung oder zum Abschminken, Körpermilch zum Schutz oder zur Pflege; Reinigungsmilch;
- desodorisierende Zusammensetzung; nach der Rasur anzuwendendes Gel oder nach der Rasur anzuwendende Lotion; Depiliercreme;
- pharmazeutische Zusammensetzung;
- feste Zusammensetzung, beispielsweise als Seife oder Reinigungsstücke;
- Zusammensetzung für Aerosole, die auch ein Treibmittel unter Druck enthält;
- Zusammensetzung für die Haarpflege, insbesondere als Haarwaschmittel, Lotion für eine Wasserwelle, Behandlungslotion, Frisiercreme, Frisiergel, Zusammensetzung zum Färben (insbesondere zum oxidativen Färben) gegebenenfalls in Form eines färbenden Haarwaschmittels, restrukturierende Lotion für das Haar, Zusammensetzung für eine permanente Verformung, Lotion oder Gel gegen Haarausfall, antiparasitäres Haarwaschmittel, Lotion oder Haarwaschmittel gegen Schuppen; Haarwaschmittel zur Behandlung und insbesondere gegen Seborrhoe;
- Zusammensetzung zur Verwendung im Mund-Zahn-Bereich, beispielsweise als Zahncreme.

## Claims

1. Process for preserving a cosmetic or pharmaceutical composition, **characterized in that** it consists in incorporating into the said composition at least one polyamino acid derivative corresponding to formula (I) : 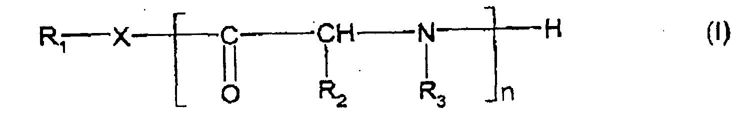 in which:
- X represents O, S, NH or NR" with R" representing a saturated or unsaturated, linear or branched C₁-C₆ hydrocarbon-based radical;
- R₁ represents
- a linear or branched, saturated or unsaturated, C₁-C₄₀ hydrocarbon-based radical, optionally substituted with at least one hydroxyl radical and/or one radical -NRR' and/or optionally interrupted with at least one heteroatom chosen from N, O and Si, R and R', which may be identical or different, being a hydrogen atom or a saturated or unsaturated, linear or branched, C₁-C₆ hydrocarbon-based radical;
- a radical 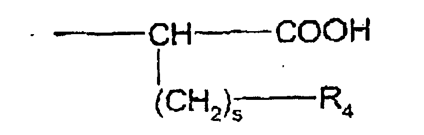 with s equal to 0, 1, 2, 3 or 4; and R₄ representing a hydrogen atom or a radical chosen from -NH₂, -OH, -SH, -CHOHCH₃, -CONH₂, -NH-C(NH₂)=NH, -C₆H₅, -C₆H₄OH and 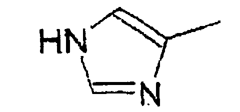
- a radical 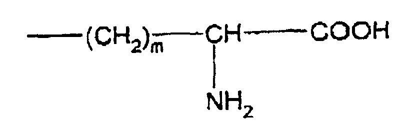 with m equal to 3, 4 or 5;
- R₂ represents a hydrogen atom; a saturated or unsaturated, linear or branched C₂-C₈ hydrocarbon-based radical; a radical chosen from -CH₂C₆H₅, -CH₂C₆H₄OH, -CH₂OH, -CHOHCH₃ and -(CH₂)ₜ-NH₂ with t equal to 3 or 5;
- R₃ represents a hydrogen atom or a saturated or unsaturated, linear or branched C₁-C₆ hydrocarbon-based radical;
- n is an average number greater than 1 such that the molecular weight of the polyamino acid derivative is between 100 and 200,000;
- it being possible for the repeating unit to be identical or different for the same derivative, R₂ and/or R₃ then taking at least one of the other meanings given for these radicals,
or a salt thereof.

2. Process according to Claim 1, in which the polyamino acid derivative corresponds to formula (I) in which:
- X represents O, S, NH or NR" with R" representing a saturated or unsaturated, linear or branched C₁-C₆ hydrocarbon-based radical;
- R₁ represents a linear or branched, saturated or unsaturated C₈-C₄₀ hydrocarbon-based radical, optionally substituted with at least one hydroxyl radical and/or one radical -NRR' and/or optionally interrupted with at least one heteroatom chosen from N, O and Si, R and R', which may be identical or different, being a hydrogen atom or a saturated or unsaturated, linear or branched, C₁-C₆ hydrocarbon-based radical;
- R₂ represents a hydrogen atom;
- R₃ represents a saturated, linear or branched, C₁-C₆ hydrocarbon-based radical; in particular a methyl or ethyl radical; and/or
- n is between 2 and 100 and/or is such that the molecular weight of the polyamino acid derivative is between 150 and 10,000.

3. Process according to either of the preceding claims, in which the polyamino acid derivative corresponds to formula (I) in which:
- X represents NH;
- R₁ represents a linear or branched, saturated C₁₀-C₂₄ hydrocarbon-based radical, optionally substituted with 1, 2, 3 or 4 hydroxyl radicals; or a linear or branched hydrocarbon-based radical comprising one or more C₁₂-C₂₄ double unsaturations, optionally substituted with at least one hydroxyl radical;
- R₂ represents a hydrogen atom;
- R₃ represents a methyl radical; and/or
- n is between 4 and 50 and/or is such that the molecular weight of the polyamino acid derivative is between 300 and 8000.

4. Process according to one of the preceding claims, in which the cosmetic or pharmaceutical composition moreover comprises a physiologically acceptable medium.

5. Process according to one of the preceding claims, in which the polyamino acid derivative is present in the cosmetic or pharmaceutical composition in an amount of between 0.001% and 30% by weight, preferably between 0.01% and 15% by weight and more particularly between 0.5% and 5% by weight, relative to the total weight of the composition.

6. Process according to one of the preceding claims, in which the cosmetic or pharmaceutical composition is:
- in the form of a makeup product for the skin of the face, of the body or of the lips, such as a foundation, a makeup rouge, an eye shadow, a concealer stick, a cover stick, an eyeliner, a mascara, a lipstick, a nail varnish or a nailcare product;
- in the form of a dermatological or cosmetic care product for the skin of the face, of the body, including the scalp, or of the lips, such as a lipcare base, a fixing base to be applied over a conventional lipstick, an antisun composition or an artificial tanning composition; a deodorant product; a care composition (day, night, anti-wrinkle, moisturizing, etc. product) for the face; a matt-effect composition for the face; a cleansing or makeup-removing gel or cream; a protective body milk or a bodycare milk; or a purifying milk;
- in the form of a deodorant composition; an aftershave gel or lotion; or a hair-removing cream;
- in the form of a pharmaceutical composition;
- in the form of a solid composition such as a cleansing soap or bar;
- in the form of an aerosol composition also comprising a propellant under pressure;
- in the form of a haircare composition, and in particular a shampoo, a hairsetting lotion, a medicated lotion, a styling cream or gel, a dye composition (in particular an oxidation dye composition) optionally in the form of a colouring shampoo, a restructuring lotion for the hair, a permanent-waving composition, a lotion or gel for preventing hair loss, an antiparasitic shampoo; an antidandruff lotion or shampoo; or a medicated shampoo, in particular an anti-seborrhoeic shampoo;
- in the form of a composition for buccodental use, for example a toothpaste.
